Europäisches Patentamt

European Patent Office

Office européen des brevets

(11) Publication number: **0 409 337 A1**

# EUROPEAN PATENT APPLICATION

(21) Application number: 90201921.5

(22) Date of filing: 16.07.90

(51) Int. Cl.5: **H01J 61/34**, C02F 1/32, H01J 5/03

(30) Priority: 21.07.89 NL 8901890

(43) Date of publication of application:
23.01.91 Bulletin 91/04

(84) Designated Contracting States:
AT BE CH DE ES FR GB IT LI NL

(71) Applicant: N.V. Philips' Gloeilampenfabrieken
Groenewoudseweg 1
NL-5621 BA Eindhoven(NL)

(72) Inventor: Oostvogels, Franciscus Martinus
Petrus
c/o INT. OCTROOIBUREAU B.V., Prof.
Holstlaan 6
NL-5656 AA Eindhoven(NL)

(74) Representative: Rooda, Hans et al
INTERNATIONAAL OCTROOIBUREAU B.V.
Prof. Holstlaan 6
NL-5656 AA Eindhoven(NL)

(54) Low-pressure metal vapour discharge lamp.

(57) The lamp has a lamp vessel (1) having a U-shaped discharge space (2). The lamp vessel is surrounded with a certain amount of clearance by a hood (4) of synthetic material, which is sealed by welding at a first end (9).

During operation, the lamp emits UV-C radiation and can be used for sterilizing narrow containers.

FIG.1

## LOW-PRESSURE METAL VAPOUR DISCHARGE LAMP.

The invention relates to a low-pressure metal vapour discharge lamp comprising
- a glass lamp vessel surrounding a discharge space and provided at one end with a lamp cap,
- a filling in the lamp vessel comprising rare gas and metal vapour,
- a hood of synthetic material surrounding the lamp with a certain amount of clearance.

Such a lamp is known from US 4,048,537 A.

The known lamp is tubular with a lamp cap at both ends and is intended to be used in chemotherapy because of the generated UV radiation. In order to prevent that in the case of rupture of the lamp vessel the environment is damaged, the lamp vessel is provided with an envelope of synthetic material. The ends of said envelope are enclosed at both ends of the tubular lamp in a respective profiled ring, which surrounds the respective end of the lamp vessel and the lamp cap mounted thereon.

A disadvantage of the known lamp is that the hood does not entirely seal the lamp and its filling with respect to the environment. Another disadvantage is that due to its shape the known lamp is not suitable to be used for irradiating a narrow space.

The invention has for its object to provide a lamp of the kind described in the opening paragraph, which has a simple construction and in which the said disadvantages are avoided.

According to the invention, this object is achieved in that
- the discharge space is U-shaped and is arranged with its ends in the lamp cap, in which the lamp vessel is fixed,
- the hood of synthetic material is fixed at a first end in the lamp cap, extends beyond the bend in the U of the discharge space and is sealed by welding at a second end.

The lamp is suitable to be inserted into bottles or other narrow containers to sterilize them before relevant articles, such as eatables and drinkables, are packed therein. The lamp cap and the hood of synthetic material together entirely enclose the lamp vessel. In the case of rupture of the lamp vessel, for example due to shocks, glass fragments or solid or liquid constituents of the filling cannot contaminate the environment. It has further been found that the hood of synthetic material surrounding the lamp vessel with a certain amount of clearance has a preventive effect with respect to rupture of the lamp vessel. It has been found on the contrary that a hood thermally shrunk around a lamp vessel induces rupture due to stresses produced in the lamp vessel.

Polymeric fluorinated hydrocarbons, such as synthetic materials of fluorinated ethene-propene, for example tetra-fluoro-ethene-hexafluoropropene-copolymer, ethene-tetrafluoro-ethene-copolymer or perfluoroalcoxy-polymer, have proved to be particularly suitable materials for the hood of synthetic material.

The lamp vessel can be held by the lamp cap with clamping fit. A rapid assembling is obtained by the use of a cement mass of, for example, cement for lamps.

Although the first end of the envelope of synthetic material may be fixed in the lamp cap in various ways, for example may be mechanically enclosed, it has found to be very practical to form a tongue at this end and to embed this tongue in a solidifying mass, for example in the cement by means of which the lamp vessel is fixed in the lamp cap.

In a favourable embodiment, the lamp vessel is sealed on the envelope of the lamp vessel by a sealing mass, for example an elastic synthetic material, such as, for example, silicone rubber. The lamp is then resistant to spraying water. The resistance to spraying water can be still further increased in that the lamp cap is also sealed by such a mass at an end remote from the lamp vessel.

In a favourable embodiment, an electrical cable is passed out of the lamp cap to be connected to a source of electric energy at an area remote from the lamp.

It is favourable if the lamp vessel consists of a glass having an $SiO_2$ content of at least 97% by weight, such as quartz glass, for a high transmission of UV radiation, and has a doping of an absorber of short- = wave UV-C radiation, such as 185 nm radiation, in order to prevent ozone formation. Such a doping, for example of 0.01% by weight of $TiO_2$, also has a favourable influence on the stability of the hood of synthetic material.

The filling of the lamp vessel may comprise besides a rare gas, such as, for example, argon, argon/neon or argon/krypton, instead of, for example, mercury an amalgam, such as Pb/Bi/Sn amalgam, in order to make the efficiency of the lamp little dependent upon temperature.

Embodiments of the lamp according to the invention are shown in the drawing. In the drawing:
Fig. 1 shows in side elevation a first embodiment, in which the lamp cap is partly broken away;
Fig. 2 shows a second embodiment in side elevation rotated with respect to Fig. 1 through 90°, in which the lamp cap is partly broken away.

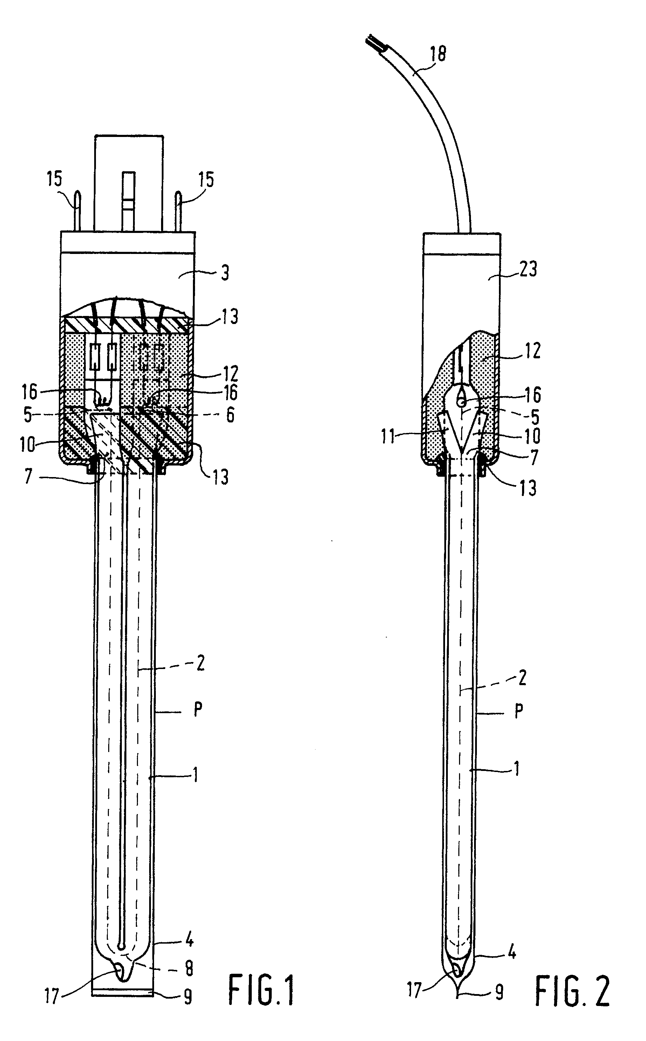

The low-pressure metal vapour discharge lamps of Figures 1 and 2 have a glass lamp vessel 1, which surrounds a discharge space 2 and is provided at one end with a lamp cap 3. The lamp vessel contains a filling, which comprises rare gas and metal vapour. A hood 4 of synthetic material surrounds with a certain amount of clearance the lamp vessel 1.

The discharge space 2 is U-shaped and is arranged with its ends 5, 6 in the lamp cap 3 and 23, respectively, in which the lamp vessel 1 is fixed. The hood 4 of synthetic material is fixed at a first end 7 in the lamp vessel, extends beyond the bend 8 in the U of the discharge space 2 and is sealed by welding at a second end 9.

The hood 4 is a hood having a thickness of about 0.4 to 1 mm, for example 0.8 mm, and consisting of polyfluoroethene-propene. At the first end 7 of the hood 4, tongues 10, 11 are provided, which are embedded in a sealing mass 13 (Fig. 1) and in a cement mass 12, respectively, (Fig. 1), for example of cement for incandescent lamps, by which the lamp vessel 1 is fixed in the lamp cap 3.

The lamp vessel 3, 23 is sealed on the hood 4 by means of a sealing mass 13, for example of silicone rubber. The lamp vessel 1 consists of glass having an $SiO_2$ content of at least 97% by weight, for example of quartz glass, doped with an absorber of short-wave UV radiation, for example with 0.02% by weight of $TiO_2$. The radiation of the lamp consequently substantially does not comprise radiation of very short wavelength, such as 185 nm, and thus substantially does no generate ozone.

The filling of the lamp vessel 1 comprises Pb/Bi/Sn amalgam 17, for example 19% by weight of Pb, 43.5% by weight of Bi, 32.5% by weight of Sn and 5% by weight of Hg, as a result of which the lamp has a substantially unchanged efficiency when the operating temperature at the place P is $80 \pm 20\,^{\circ}C$.

Electrodes 14 are arranged in the lamp vessel 1. The lamp cap 3 is sealed with respect to the lamp vessel 1 by silicone rubber 13.

The lamp cap 3 of Fig. 1 has contact pins 15 for connection to a source of supply. In Fig. 2, a cable 18 is passed out of the lamp cap to make contact with the source of supply at a certain distance from the lamp.

The lamps shown mainly emit 254 nm radiation and are suitable to be used for sterilizing, for example, glass, plastizized paper or synthetic material packings of, for example, perishables.

## Claims

1. A low-pressure metal vapour discharge lamp comprising
- a glass lamp vessel surrounding a discharge space and provided at one end with a lamp cap,
- a filling in the lamp vessel comprising rare gas and metal vapour,
- a hood of synthetic material surrounding the lamp with a certain amount of clearance, characterized in that
- the discharge space is U-shaped and is arranged with its ends in the lamp cap, in which the lamp vessel is fixed,
- the hood of synthetic material is fixed at a first end in the lamp cap, extends beyond the bend in the U of the discharge space and is sealed by welding at a second end.

2. A low-pressure discharge lamp as claimed in Claim 1, characterized in that at the first end of the hood of synthetic material a tongue is provided, which is fixed in a solidifying mass in the lamp cap.

3. A low-pressure discharge lamp as claimed in Claim 1 or 2, characterized in that the lamp cap is sealed on the hood by a sealing mass.

4. A low-pressure discharge lamp as claimed in Claim 3, characterized in that the lamp cap is sealed at an end remote from the lamp vessel.

5. A low-pressure discharge lamp as claimed in Claim 3 or 4, characterized in that an electrical cable is passed out of the lamp cap.

6. A low-pressure discharge lamp as claimed in Claim 1, characterized in that the glass of the lamp vessel has an $SiO_2$ content of at least 97% by weight and is doped with an absorber of short-wave UV-C radiation.

7. A low-pressure discharge lamp as claimed in Claim 1, characterized in that the filling comprises Pb-Bi-Sn amalgam.

EP 0 409 337 A1

FIG.1

FIG.2

4

## EUROPEAN SEARCH REPORT

European Patent Office

Application Number

EP 90 20 1921

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|----------|-------------------------------------------------------------------------------|-------------------|------------------------------------------------|
| A | EP-A-0 202 781 (W.M. STILL & SONS) <br> * Page 3, lines 11-21; figure 1 * | 1 | H 01 J 61/34 <br> C 02 F 01/32 <br> H 01 J 05/03 |
| D,A | US-A-4 048 537 (R. BLAISDELL et al.) <br> * Column 2, lines 37-43; figure 3 * | 1 | |

TECHNICAL FIELDS SEARCHED (Int. Cl.5)

H 01 J 61/00
C 02 F 1/00
H 01 J 5/00

The present search report has been drawn up for all claims

| Place of search | Date of completion of the search | Examiner |
|-----------------|----------------------------------|----------|
| THE HAGUE | 16-10-1990 | ROWLES K.E.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 01.82 (P0401)